(19)

Europäisches Patentamt

European Patent Office

Office européen des brevets

(11) **EP 4 361 289 A1**

(12) **EUROPEAN PATENT APPLICATION**
published in accordance with Art. 153(4) EPC

(43) Date of publication:
**01.05.2024 Bulletin 2024/18**

(21) Application number: **22826585.6**

(22) Date of filing: **05.09.2022**

(51) International Patent Classification (IPC):
***C12Q 1/6886*** (2018.01)

(52) Cooperative Patent Classification (CPC):
**C12Q 1/6886;** C12Q 2545/00; C12Q 2561/113;
C12Q 2600/112; C12Q 2600/118; C12Q 2600/158

(86) International application number:
**PCT/JP2022/033204**

(87) International publication number:
**WO 2024/052948 (14.03.2024 Gazette 2024/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Kubix Inc.**
**Hakusan-shi, Ishikawa 920-2161 (JP)**

(72) Inventors:
• **KANEKO Shuichi**
**Kanazawa-shi**
**Ishikawa**
**9208640 (JP)**
• **SAKAI Yoshio**
**Kanazawa-shi**
**Ishikawa**
**9208640 (JP)**

• **TANNO Hiroshi**
**Hakusan-shi**
**Ishikawa**
**9202161 (JP)**
• **SENOURA Takeshi**
**Hakusan-shi**
**Ishikawa**
**9202161 (JP)**

(74) Representative: **D Young & Co LLP**
**3 Noble Street**
**London EC2V 7BQ (GB)**

Remarks:
The complete document including Reference
Table(s) and the Sequence Listing(s) can be
downloaded from the EPO website

(54) **DETECTION OF GENE EXPRESSION PATTERN SPECIFIC TO PANCREATIC CANCER, AND DETECTION OF PANCREATIC CANCER THROUGH COMBINATION WITH MEASUREMENT OF CA19-9**

(57) This invention provides a method for detecting pancreatic cancer by analyzing a gene whose expression changes in relation to pancreatic cancer and by measuring CA19-9 in a biological sample.

It provides a method for assisting detection of pancreatic cancer based on a pancreatic cancer-specific expression level of 56 genes in a 56-gene set A and a measured value of CA19-9 in a subject.

EP 4 361 289 A1

**Description**

Technical Field

The present invention is a diagnostic aid for pancreatic cancer by detecting a pancreatic cancer-specific gene expression pattern with peripheral blood used as a material, and by simultaneously measuring CA19-9 in the blood.

Background Art

Pancreatic cancer is a malignancy of the digestive system, ranking fifth in terms of the number of cancer deaths by site in Japan (males and females combined). According to Cancer Information Service provided by National Cancer Center Japan, 37,677 patients die annually from pancreatic cancer (2020). Detection of pancreatic cancer is extremely difficult and it is rarely detected in its early stages. It is a digestive system cancer with an extremely poor prognosis wherein 75% of the cases diagnosed with pancreatic cancer are inoperable and die within 1 to 2 years after the detection (according to Institute for Cancer Control, National Cancer Center Japan. http://ganjoho.jp/public/cancer/data/pancreas.html). It has been a long time since an advancement of a diagnostic technology for pancreatic cancer was desired but no useful method for an early diagnosis has yet been established.

In recent years, the advancement in gene analysis technologies including DNA microarray technology and next-generation sequencers has made it possible to conduct a comprehensive gene expression analysis targeting all genes. This made it possible to diagnose and predict a prognosis of a new cancer, predict a recurrence rate following treatment, and the like. The group of the present inventors analyzed gene expression profiles in pancreatic cancer, developed a specific detection method for pancreatic cancer by 56-gene expression profiles (see Patent Literature 1) and a detection method using real-time PCR, and applied for and obtained a marketing authorization approval from the government.

Citation List

Patent Literature

Patent Literature 1: JP Patent No. 5852759 Description

Summary of Invention

Technical Problem

An object of the present invention is to provide a method for detecting pancreatic cancer by analyzing genes whose expression changes in relation to pancreatic cancer and by simultaneously measuring CA19-9 in a biological sample, and a detection reagent for detecting pancreatic cancer, with a method which is minimally invasive to a patient and easy to extract genes from the patient.

Solution to Problem

In view of the fact that a detection method by real-time PCR can be performed at a lower cost than a detection method using DNA microarray and allows for more rapid detection, the present inventors analyzed gene expression profiles of patients with pancreatic cancer for developing a method of detecting pancreatic cancer by real-time PCR. Furthermore, they simultaneously measured CA19-9, which is a tumor marker with relatively high sensitivity in pancreatic cancer detection, and evaluated the usefulness of combining results of real-time PCR and results of CA19-9 measurement in detecting pancreatic cancer.

As a result, the inventors found out a 56-gene set A associated with pancreatic cancer, further conducted a statistical discriminant analysis of relationship between the expression level of these genes of the set and pancreatic cancer, and found that pancreatic cancer can be detected with a high sensitivity by combining the results of the expression level of the 56 genes and the measured value of CA19-9, thus completing the present invention.

Specifically, the present invention is summarized as follows.

[1] A method for assisting detection of pancreatic cancer based on an expression level of 56 genes in a 56-gene set A listed below and a measured value of CA19-9 in a subject:
the gene set A being

(1) Abhydrolase domain containing 3 (ABHD3)

(2) Abl-interactor 1 (ABI1)

(3) Acyl-CoA synthetase long-chain family member 3 (ACSL3)

(4) Aldo-keto reductase family 1, member B1 (aldose reductase) (AKR1B 1)

(5) ATPase, Class VI, type 11B (ATP11B)

(6) UDP-GlcNAc:betaGal beta-1,3-N-acetylglucosaminyltransferase 5 (B3GNT5)

(7) BTB and CNC homology 1, basic leucine zipper transcription factor 1 (BACH1)

(8) Chromosome 11 open reading frame 2 (C11orf2)

(9) Chromosome 2 open reading frame 81 (C2orf81)

(10) Cyclin Y-like 1 (CCNYL1)

(11) Centromere protein N (CENPN)

(12) Complement factor H-related 3 (CFHR3)

(13) C-type lectin domain family 4, member D (CLEC4D)

(14) Collagen, type XVII, alpha 1 (COL17A1)

(15) Cytochrome b5 reductase 4 (CYB5R4)

(16) DENN/MADD domain containing 1B (DENND1B)

(17) Enoyl CoA hydratase domain containing 3 (ECHDC3)

(18) Ectonucleoside triphosphate diphosphohydrolase 1 (ENTPD1)

(19) Family with sequence similarity 198, member B (FAM198B)

(20) Family with sequence similarity 49, member B (FAM49B)

(21) Fatty acyl CoA reductase 1 (FAR1)

(22) Fibrinogen-like 2 (FGL2)

(23) Fibronectin type III domain containing 3B (FNDC3B)

(24) Fucose-1-phosphate guanylyltransferase (FPGT)

(25) UDP-N-acetyl-alpha-D-galactosamine:polypeptide N-acetylgalactosaminyltransferase 4 (GalNAc-T4) (GALNT4)

(26) HEAT repeat containing 5A (HEATR5A)

(27) HIV-1 Tat interactive protein 2, 30kDa (HTATIP2)

(28) Interferon gamma receptor 1 (IFNGR1)

(29) IKBKB interacting protein (IKBIP)

(30) Lactate dehydrogenase A (LDHA)

(31) Lysophosphatidic acid receptor 6 (LPAR6)

(32) Membrane-bound transcription factor peptidase, site 2 (MBTPS2)

(33) Minichromosome maintenance complex binding protein (MCMBP)

(34) Mesoderm induction early response 1 homolog (Xenopus laevis) (MIER1)

(35) Nuclear factor (erythroid-derived 2) -like 2 (NFE2L2)

(36) Oligonucleotide/oligosaccharide-binding fold containing 2A (OBFC2A)

(37) Oxysterol binding protein-like 8 (OSBPL8)

(38) Peptidylprolyl isomerase H (cyclophilin H) (PPIH)

(39) Protein phosphatase 1, regulatory (inhibitor) subunit 13 like (PPP1R13L)

(40) PR domain containing 5 (PRDM5)

(41) Protein tyrosine phosphatase, receptor type, C (PTPRC)

(42) RAB10, member RAS oncogene family (RAB10)

(43) Ribosomal protein, large, P1 (RPLP1)

(44) Ras-related GTP binding D (RRAGD)

(45) Solute carrier family 22, member 15 (SLC22A15)

(46) Solute carrier family 44, member 1 (SLC44A1)

(47) Schlafen family member 12 (SLFN12)

(48) S1 RNA binding domain 1 (SRBD1)

(49) Tet oncogene family member 2 (TET2)

(50) Transducin-like enhancer of split 2 (E(sp1) homolog, Drosophila) (TLE2)

(51) Ubiquitin-conjugating enzyme E2W (putative) (UBE2W)

(52) Ubiquitination factor E4A (UBE4A)

(53) Ubiquitin specific peptidase 15 (USP15)

(54) WD repeat and SOCS box containing 1 (WSB1)

(55) Zinc finger E-box binding homeobox 2 (ZEB2)

(56) Zinc metallopeptidase (STE24 homolog, S. cerevisiae) (ZMPSTE24)

[2] The method of [1], wherein the expression level of the genes in the subject is measured using mRNA of peripheral blood cells of the subject.

[3] The method of [1], wherein the gene expression level is measured by quantitative PCR targeting a nucleotide consisting of a nucleotide sequence of the 56 genes in the 56-gene set A according to claim 1 or a nucleotide comprising a partial nucleotide sequence thereof, and used in combination with the measured value of CA19-9.

[4] The method of any one of [1] to [3], comprising steps below:

(1) a step of extracting mRNA from the peripheral blood cells collected from the subject;

(2) a step of amplifying the 56 genes in the gene set A by PCR to obtain a Ct (Cycle threshold) value, and standardizing it with a Ct value of a housekeeping gene such as GAPDH to obtain a standardized Ct value;

(3) a step of measuring CA19-9 in a biological sample collected from the subject;

(4) a step of calculating a value for assisting in determining whether it is positive or negative for pancreatic cancer from the standardized Ct Value of each gene in the gene set A and the measured value of CA19-9;

[5] The method of [4], comprising steps below wherein if a final determination value Z is greater than 0, it is determined positive for pancreatic cancer:

(1) a step of calculating a P value from the standardized Ct value obtained in [4] step (2) using a discriminant formula I described below,

$$\text{Discriminant formula I: intercept} + \sum (\text{beta } i \times X \, i)$$

wherein intercept is a constant, beta i is a coefficient for the i-th gene of the 56 genes in the gene set A, and x i indicates summing beta × X for each of the 56 genes in the gene set A; and

(2) a step of setting the measured value of CA19-9 obtained in [4] step (3) as a C value and assigning the P value and the C value to a discriminant formula II described below to obtain a final determination value Z,

$$\text{Discriminant formula II: } -2.9448 + (0.0090864 \times P) + (0.9329593 \times \text{Loge C}).$$

[6] The method for detecting pancreatic cancer of [5], wherein the values of intercept and beta i for each gene in the discriminant formula using the 56-gene set A are as follows: intercept: -298.018

Set A

| No. | Gene name | beta |
|-----|-----------|----------|
| 1 | ABHD3 | 18.25234 |
| 2 | ABI1 | 5.533139 |
| 3 | ACSL3 | -8.30246 |
| 4 | AKR1B1 | 8.005092 |
| 5 | ATP 11B | -16.3131 |
| 6 | B3GNT5 | -12.6434 |
| 7 | BACH1 | 12.79754 |
| 8 | C11orf2 | -8.85243 |
| 9 | C2orf81 | 8.747677 |
| 10 | CCNYL1 | 6.300126 |
| 11 | CENPN | 4.732161 |
| 12 | CFHR3 | -6.547 |

(continued)

| No. | Gene name | beta |
|-----|-----------|----------|
| 13 | CLEC4D | -7.20897 |
| 14 | COL17A1 | 7.970873 |
| 15 | CYB5R4 | -11.661 |
| 16 | DENND 1B | -10.4299 |
| 17 | ECHDC3 | -8.66068 |
| 18 | ENTPD1 | -7.19342 |
| 19 | FAM198B | -13.2931 |
| 20 | FAM49B | -8.78217 |
| 21 | FAR1 | 7.238576 |
| 22 | FGL2 | -8.25242 |
| 23 | FNDC3B | 14.19903 |
| 24 | FPGT | -8.48534 |
| 25 | GALNT4 | 6.227974 |
| 26 | HEATR5A | 5.784991 |
| 27 | HTATIP2 | 12.98543 |
| 28 | IFNGR1 | -6.31219 |
| 29 | IKBIP | -4.35974 |
| 30 | LDHA | 13.84082 |
| 31 | LPAR6 | -11.9776 |
| 32 | MBTPS2 | 11.32147 |
| 33 | MCMBP | 7.570634 |
| 34 | MIER1 | 4.196767 |
| 35 | NFE2L2 | 11.75957 |
| 36 | OBFC2A | -11.0293 |
| 37 | OSBPL8 | 6.956427 |
| 38 | PPIH | 5.40687 |
| 39 | PPP1R13L | 8.60306 |
| 40 | PRDM5 | -8.77508 |
| 41 | PTPRC | 15.18565 |
| 42 | RAB10 | 13.27343 |
| 43 | RPLP 1 | 13.75907 |
| 44 | RRAGD | 6.236001 |
| 45 | SLC22A15 | -5.63841 |
| 46 | SLC44A1 | 18.88705 |
| 47 | SLFN12 | -7.8338 |
| 48 | SRBD 1 | -12.4726 |
| 49 | TET2 | 11.42427 |
| 50 | TLE2 | 14.37697 |

(continued)

| No. | Gene name | beta |
|-----|-----------|-----------|
| 51 | UBE2W | 5.881697 |
| 52 | UBE4A | 5.612217 |
| 53 | USP15 | -7.21872 |
| 54 | WSB1 | -13.4219 |
| 55 | ZEB2 | -8.42855 |
| 56 | ZMPSTE24 | 6.555173 |

[7] A reagent for detecting pancreatic cancer, comprising a nucleotide consisting of a nucleotide sequence of the genes in the 56-gene set A or a nucleotide comprising a partial nucleotide sequence thereof for detecting pancreatic cancer by use in combination with the measurement of CA 19-9:

Gene set A

(1) Abhydrolase domain containing 3 (ABHD3)
(2) Abl-interactor 1 (ABI1)
(3) Acyl-CoA synthetase long-chain family member 3 (ACSL3)
(4) Aldo-keto reductase family 1, member B1 (aldose reductase) (AKR1B 1)
(5) ATPase, Class VI, type 11B (ATP11B)
(6) UDP-GlcNAc:betaGal beta-1,3-N-acetylglucosaminyltransferase 5 (B3GNT5)
(7) BTB and CNC homology 1, basic leucine zipper transcription factor 1 (BACH1)
(8) Chromosome 11 open reading frame 2 (C11orf2)
(9) Chromosome 2 open reading frame 81 (C2orf81)
(10) Cyclin Y-like 1 (CCNYL1)
(11) Centromere protein N (CENPN)
(12) Complement factor H-related 3 (CFHR3)
(13) C-type lectin domain family 4, member D (CLEC4D)
(14) Collagen, type XVII, alpha 1 (COL17A1)
(15) Cytochrome b5 reductase 4 (CYB5R4)
(16) DENN/MADD domain containing 1B (DENND1B)
(17) Enoyl CoA hydratase domain containing 3 (ECHDC3)
(18) Ectonucleoside triphosphate diphosphohydrolase 1 (ENTPD1)
(19) Family with sequence similarity 198, member B (FAM198B)
(20) Family with sequence similarity 49, member B (FAM49B)
(21) Fatty acyl CoA reductase 1 (FAR1)
(22) Fibrinogen-like 2 (FGL2)
(23) Fibronectin type III domain containing 3B (FNDC3B)
(24) Fucose-1-phosphate guanylyltransferase (FPGT)
(25)  UDP-N-acetyl-alpha-D-galactosamine:polypeptide  N-acetylgalactosaminyltransferase  4  (GalNAc-T4) (GALNT4)
(26) HEAT repeat containing 5A (HEATR5A)
(27) HIV-1 Tat interactive protein 2, 30kDa (HTATIP2)
(28) Interferon gamma receptor 1 (IFNGR1)
(29) IKBKB interacting protein (IKBIP)
(30) Lactate dehydrogenase A (LDHA)
(31) Lysophosphatidic acid receptor 6 (LPAR6)
(32) Membrane-bound transcription factor peptidase, site 2 (MBTPS2)
(33) Minichromosome maintenance complex binding protein (MCMBP)
(34) Mesoderm induction early response 1 homolog (Xenopus laevis) (MIER1)
(35) Nuclear factor (erythroid-derived 2) -like 2 (NFE2L2)
(36) Oligonucleotide/oligosaccharide-binding fold containing 2A (OBFC2A)

(37) Oxysterol binding protein-like 8 (OSBPL8)
(38) Peptidylprolyl isomerase H (cyclophilin H) (PPIH)
(39) Protein phosphatase 1, regulatory (inhibitor) subunit 13 like (PPP1R13L)
(40) PR domain containing 5 (PRDM5)
(41) Protein tyrosine phosphatase, receptor type, C (PTPRC)
(42) RAB10, member RAS oncogene family (RAB10)
(43) Ribosomal protein, large, P1 (RPLP1)
(44) Ras-related GTP binding D (RRAGD)
(45) Solute carrier family 22, member 15 (SLC22A15)
(46) Solute carrier family 44, member 1 (SLC44A1)
(47) Schlafen family member 12 (SLFN12)
(48) S1 RNA binding domain 1 (SRBD1)
(49) Tet oncogene family member 2 (TET2)
(50) Transducin-like enhancer of split 2 (E(sp1) homolog, Drosophila) (TLE2)
(51) Ubiquitin-conjugating enzyme E2W (putative) (UBE2W)
(52) Ubiquitination factor E4A (UBE4A)
(53) Ubiquitin specific peptidase 15 (USP15)
(54) WD repeat and SOCS box containing 1 (WSB1)
(55) Zinc finger E-box binding homeobox 2 (ZEB2)
(56) Zinc metallopeptidase (STE24 homolog, S. cerevisiae) (ZMPSTE24)

[8] The reagent of [7], wherein the nucleotide comprising the partial nucleotide sequence is a primer for PCR.

Advantageous Effects of Invention

Based on the expression level of genes of the gene set in the 56-gene set A measured by real-time PCR of the present invention, whether a subject has pancreatic cancer or not can be determined with high accuracy. Specifically, by setting the expression level of the 56 genes in the gene set A developed by statistical analysis as a variable and introducing the CA19-9 value simultaneously measured into the special discriminant formulas, pancreatic cancer can be detected easily and with high accuracy.

Brief Description of Drawings

[Figure 1] Figure 1 shows a correlation between C value (measured value of CA19-9) and P value (calculated value of the discriminant formula based on the result of gene analysis).
[Figure 2] Figure 2 shows sensitivity and specificity in the case of detecting pancreatic cancer using CA19-9 alone and in the case of detecting pancreatic cancer using the measured value of CA19-9 in combination with the calculated value by the 56-gene expression analysis.
[Figure 3] Figure 3 shows sensitivity by clinical stage (Stage I/II and Stage III/IV) in the case of detecting pancreatic cancer using CA19-9 alone and in the case of using the measured value of CA19-9 in combination with the calculated value by the 56-gene expression analysis.

Description of Embodiments

Hereinafter, the present invention is described in detail.

1. Detection of pancreatic cancer by combining gene expression analysis and CA19-9 measurement

(i) Gene expression analysis

Genes used for methods of the present invention are 56 genes whose expression level changes in pancreatic cancer. An expression level of these 56 genes can be measured to analyze their expression profile.

The genes used for the methods of the present invention can be selected by a method described below. Patients diagnosed by a physician as having pancreatic cancer are assigned to a group of pancreatic cancer patients. Peripheral blood is collected from the group of pancreatic cancer patients and a group of healthy subjects, and total mRNA is isolated from the peripheral blood. The isolated mRNA is applied to DNA microarray containing human genes to measure the expression level of the genes, and a hierarchical clustering analysis is performed to select genes having different expression levels between the group of pancreatic cancer patients and the group of healthy subjects. Real-time PCR is used to perform an expression analysis of the genes selected by the microarray analysis for the group of pancreatic cancer patients and the group of healthy subjects. In this case, the expression can be analyzed with a housekeeping gene such as GAPDH (glyceraldehyde 3-phosphate dehydrogenase) as a control. Thus, by using real-time PCR analysis, genes from the group of pancreatic cancer patients whose expression level differs from the group of healthy subjects can be finally selected as pancreatic cancer-specific genes.

The genes used in the present invention selected by the above method are 56 genes listed below. Symbol, NCBI RefSeq ID and SEQ ID NO of the genes are shown in parentheses.

(1) Abhydrolase domain containing 3 (ABHD3) (NM_138340.4) (SEQ ID No. 1)

(2) Abl-interactor 1 (ABI1) (NM_005470.3) (SEQ ID No. 2)

(3) Acyl-CoA synthetase long-chain family member 3 (ACSL3) (NM_004457.3) (SEQ ID No. 3)

(4) Aldo-keto reductase family 1, member B1 (aldose reductase) (AKR1B 1) (NM_001628.2) (SEQ ID No. 4)

(5) ATPase, Class VI, type 11B (ATP11B) (NM_014616.2) (SEQ ID No. 5)

(6) UDP-GlcNAc:betaGal beta-1,3-N-acetylglucosaminyltransferase 5 (B3GNT5) (NM_032047.4) (SEQ ID No. 6)

(7) BTB and CNC homology 1, basic leucine zipper transcription factor 1 (BACH1) (NM_00 1186.3) (SEQ ID No. 7)

(8) Chromosome 11 open reading frame 2 (C11orf2) (NM_013265.3) (SEQ ID No. 8) (also called as vacuolar protein sorting 51 homolog (S. cerevisiae) (VPS51))

(9) Chromosome 2 open reading frame 81 (C2orf81) (NM_001145054.1) (SEQ ID No. 9)

(10) Cyclin Y-like 1 (CCNYL1) (NM_152523.2) (SEQ ID No. 10)

(11) Centromere protein N (CENPN) (NM_018455.5) (SEQ ID No. 11)

(12) Complement factor H-related 3 (CFHR3) (NM_021023.5) (SEQ ID No. 12)

(13) C-type lectin domain family 4, member D (CLEC4D) (NM_080387.4) (SEQ ID No. 13)

(14) Collagen, type XVII, alpha 1 (COL17A1) (NM_000494.3) (SEQ ID No. 14)

(15) Cytochrome b5 reductase 4 (CYB5R4) (NM_016230.3) (SEQ ID No. 15)

(16) DENN/MADD domain containing 1B (DENND1B) (NM_001195215.1) (SEQ ID No. 16)

(17) Enoyl CoA hydratase domain containing 3 (ECHDC3) (NM_024693.4) (SEQ ID No. 17)

(18) Ectonucleoside triphosphate diphosphohydrolase 1 (ENTPD1) (NM_001776.5) (SEQ ID No. 18)

(19) Family with sequence similarity 198, member B (FAM198B) (NM_016613.6) (SEQ ID No. 19)

(20) Family with sequence similarity 49, member B (FAM49B) (NM_016623.4) (SEQ ID No. 20)

(21) Fatty acyl CoA reductase 1 (FAR1) (NM_032228.5) (SEQ ID No. 21)

(22) Fibrinogen-like 2 (FGL2) (NM_006682.2) (SEQ ID No. 22)

(23) Fibronectin type III domain containing 3B (FNDC3B) (NM_022763.3) (SEQ ID No. 23)

(24) Fucose-1-phosphate guanylyltransferase (FPGT) (NM_003838.4) (SEQ ID No. 24)

(25) UDP-N-acetyl-alpha-D-galactosamine:polypeptide N-acetylgalactosaminyltransferase 4 (GalNAc-T4) (GALNT4) (NM_003774.4) (SEQ ID No. 25)

(26) HEAT repeat containing 5A(HEATR5A) (NM_015473.3) (SEQ ID No. 26)

(27) HIV-1 Tat interactive protein 2, 30kDa (HTATIP2) (NM_006410.4) (SEQ ID No. 27)

(28) Interferon gamma receptor 1 (IFNGR1) (NM_000416.2) (SEQ ID No. 28)

(29) IKBKB interacting protein (IKBIP) (NM_201612.2) (SEQ ID No. 29)

(30) Lactate dehydrogenase A (LDHA) (NM_005566.3) (SEQ ID No. 30)

(31) Lysophosphatidic acid receptor 6 (LPAR6) (NM_005767.5) (SEQ ID No. 31)

(32) Membrane-bound transcription factor peptidase, site 2 (MBTPS2) (NM_015884.3) (SEQ ID No. 32)

(33) Minichromosome maintenance complex binding protein (MCMBP) (NM_024834.3) (SEQ ID No. 33)

(34) Mesoderm induction early response 1 homolog (Xenopus laevis) (MIER1) (NM_020948.3) (SEQ ID No. 34)

(35) Nuclear factor (erythroid-derived 2) -like 2 (NFE2L2) (NM_006164.4) (SEQ ID No. 35)

(36) Oligonucleotide/oligosaccharide-binding fold containing 2A (OBFC2A) (NM_001031716.2) (SEQ ID No. 36) (also called as nucleic acid binding protein 1 (NABP1))

(37) Oxysterol binding protein-like 8 (OSBPL8) (NM_020841.4) (SEQ ID No. 37)

(38) Peptidylprolyl isomerase H (cyclophilin H) (PPIH) (NM_006347.3) (SEQ ID No. 38)

(39) Protein phosphatase 1, regulatory (inhibitor) subunit 13 like (PPP1R13L) (NM_006663.3) (SEQ ID No. 39)

(40) PR domain containing 5 (PRDM5) (NM_018699.3) (SEQ ID No. 40)

(41) Protein tyrosine phosphatase, receptor type, C (PTPRC) (NM_002838.4) (SEQ ID No. 41)

(42) RAB10, member RAS oncogene family (RAB10) (NM_016131.4) (SEQ ID No. 42)

(43) Ribosomal protein, large, P1 (RPLP1) (NM_001003.2) (SEQ ID No. 43)

(44) Ras-related GTP binding D (RRAGD) (NM_021244.4) (SEQ ID No. 44)

(45) Solute carrier family 22, member 15 (SLC22A15) (NM_018420.2) (SEQ ID No. 45)

(46) Solute carrier family 44, member 1 (SLC44A1) (NM_080546.4) (SEQ ID No. 46)

(47) Schlafen family member 12 (SLFN12) (NM_018042.4) (SEQ ID No. 47)

(48) S1 RNA binding domain 1 (SRBD1) (NM_018079.4) (SEQ ID No. 48)

(49) Tet oncogene family member 2 (TET2) (NM_017628.4) (SEQ ID No. 49)

(50) Transducin-like enhancer of split 2 (E(sp1) homolog, Drosophila) (TLE2) (NM_003260.4) (SEQ ID No. 50)

(51) Ubiquitin-conjugating enzyme E2W (putative) (UBE2W) (NM_018299.4) (SEQ ID No. 51)

(52) Ubiquitination factor E4A (UBE4A) (NM_004788.3) (SEQ ID No. 52)

(53) Ubiquitin specific peptidase 15 (USP15) (NM_006313.2) (SEQ ID No. 53)

(54) WD repeat and SOCS box containing 1 (WSB1) (NM_015626.8) (SEQ ID No. 54)

(55) Zinc finger E-box binding homeobox 2 (ZEB2) (NM_014795.3) (SEQ ID No. 55)

(56) Zinc metallopeptidase (STE24 homolog, S. cerevisiae) (ZMPSTE24) (NM_005857.4) (SEQ ID No. 56)

Nucleotide sequences of the 56 genes (1) to (56) above are shown in SEQ ID NO:1 to SEQ ID NO:56 in the Sequence Listing, respectively.

A nucleotide used in the methods of the present invention includes a nucleotide comprising the nucleotide sequence above or a nucleotide consisting of the fragment nucleotide sequence thereof. The nucleotide used in the present invention also includes a nucleotide which hybridizes under stringent conditions with a nucleotide having the nucleotide sequence set forth in the SEQ ID NOs above and a nucleotide consisting of the fragment sequence thereof. Examples of such a nucleotide include a nucleotide comprising a nucleotide sequence which has an overall average degree of sequence identity with the above nucleotide sequence of about 80% or more, preferably about 90% or more, more preferably about 95% or more, and especially preferably about 98% or more. Hybridization can be performed according to methods known in the art, such as those described in Current Protocols in Molecular Biology (edited by Frederick M. Ausubel et al., 1987), or other methods equivalent thereto. When a commercially available library is used, hybridization can also be performed according to a method described in the attached instructions for use. Here, "stringent conditions" are, for example, conditions equivalent to "1 XSSC, 0.1% SDS, 37°C," more stringent conditions are conditions equivalent to "0.5 XSSC, 0.1% SDS, 42°C," and further more stringent conditions are conditions equivalent to "0.2 XSSC, 0.1% SDS, 65°C." Thus, as the hybridization conditions become more stringent, a nucleotide having a higher sequence identity with the probe sequence can be isolated. However, the combinations of SSC, SDS and temperature above are examples. Those skilled in the art can achieve a stringency similar to the above by appropriately combining the above or other factors which determine hybridization stringency (such as probe concentration, probe length, hybridization reaction time). In addition, these genes may have variants, and the genes used in the present invention include variants of the above genes. Nucleotide sequences of variants can be obtained by accessing gene databases. The nucleotide of the present invention includes a nucleotide comprising a nucleotide sequence of the variant and a nucleotide consisting of a fragment sequence thereof.

In addition, the nucleotide used in the present invention can be either a nucleotide comprising a sense strand or a nucleotide comprising an antisense strand of the above genes.

In the present invention, the term gene expression level refers to the amount, intensity or frequency of gene expression, and can typically be analyzed by the amount of a transcript product produced corresponding to the gene, or the amount or activity of a translation product produced thereof. The term expression profile refers to information on the expression level of each gene. The gene expression level may be expressed as an absolute value or as a relative value. In some cases, the expression profile is also referred to as an expression pattern.

Measurement of the expression level may be performed by measuring a transcript product of a gene, i.e., mRNA, or by measuring a translation product of a gene, i.e., protein. Preferably the measurement is performed by measuring the transcript product of a gene. The transcript product of a gene also includes cDNA obtained by reverse transcription from mRNA.

To measure a transcript product of a gene, a nucleotide comprising all or part of the nucleotide sequences of the above genes can be used as a probe or primer to measure the degree of the gene expression. The degree of the gene expression can be measured by a quantitative PCR method targeting a gene or its fragment to be quantified, a method using microarray (microchip), a Northern blotting, and the like. Preferably, the gene expression is measured by the quantitative PCR method.

Examples of quantitative PCR method include agarose gel electrophoresis, fluorescent probe method, RT-PCR method, real-time PCR method, ATAC-PCR method (Kato, K. et al., Nucl. Acids Res.,25, 4694-4696, 1997), Taqman PCR method (SYBR(R) Green method), (Schmittgen TD, Methods 25, 383-385, 2001), Body Map method (Gene, 174, 151-158 (1996)), Serial analysis of gene expression (SAGE) method (US Patent No.527,154, No.544,861, European Patent Publication No.0761822), and MAGE method (Micro-analysis of Gene Expression) (JP Patent Publication (Kokai) No.2000-232888 (2000)). Examples of real-time PCR include a method using a TaqMan(R) probe. Any of the methods listed here can be performed by a known method.

PCR method can be performed by a known method. The length of a primer to be used is from 5 to 50 nucleotides, preferably from 10 to 30 nucleotides, and more preferably from 15 to 25 nucleotides. Typically, a forward primer and a reverse primer are designed based on the nucleotide sequence of the target gene. In the methods of the present invention, the sequence of the primer can be designed based on the nucleotide sequence of the target genes of SEQ ID NO:1 to SEQ ID NO:56.

The present invention comprises a reagent for detecting pancreatic cancer used in combination with the measurement of CA19-9 comprising a nucleotide consisting of a nucleotide sequence of the genes in the 56-gene set A or a nucleotide comprising a partial sequence thereof. The reagent is, for example, a primer for PCR.

The amount of messenger RNA (mRNA) transcribed from all or part of the above genes can be measured using these methods.

In the case of measuring the transcript product of the genes, the sample can be a cell of the subject and mRNA can be extracted and isolated from the cell. The cell is preferably a cell in the blood, although it is not limited thereto.

(ii) Measurement of CA-19-9

CA-19-9 included in a biological sample can be measured by immunoassay.

Examples of the biological sample provided for the present invention include serum.

Examples of the immunoassay include enzyme immunoassay (EIA), ELISA, radioimmunoassay (RIA), chemiluminescent immunoassay, immunochromatography, hemagglutination test, latex method, immunodiffusion method, immunoturbidimetric method, and immunonephelometry. Preferably, chemiluminescent immunoassay is to be used. Measurement of CA19-9 can be performed with a commercially available measurement kit. An example thereof is Chemilumi ACS Centaur CA19-9II from LSI Medience Corporation. In this case, an automatic analyzer may be used. The automatic analyzer is a biochemical autoanalyzer applicable to immunoassay. Various analyzers of this kind are commercially available. Examples thereof include ADVIA Centaur XP from Siemens Healthcare Diagnostics K.K.

2. Detection of pancreatic cancer

The methods of the present invention can be used as a diagnostic aid for diagnosing that a subject has pancreatic cancer.

For example, in a subject, among the above 56-gene set A, if the expression of a gene which increases in pancreatic cancer patients increases, or if the expression of a gene which diminishes in pancreatic cancer patients diminishes, or if the expression of a gene which increases in pancreatic cancer patients increases and the expression of a gene which diminishes in pancreatic cancer patients diminishes, whether the subject has pancreatic cancer or not can be evaluated and determined according to the value obtained by the determination formula regardless of the measured value of CA19-9 in the biological sample. In the present invention, the above evaluation and determination of whether the subject has pancreatic cancer or not is broadly referred to as detection of pancreatic cancer.

In addition, by obtaining all gene expression profiles of the above 56-gene set A to analyze the expression profile, and further obtaining the measured value of CA19-9, the gene expression profile can be combined with the measured value of CA19-9 to evaluate and determine a pathological condition of the subject. If the expression profile obtained from the subject is similar to those obtained in the group of pancreatic cancer patients, and if the value of the determination formula indicates it is positive for pancreatic cancer, regardless of the CA19-9 value, the subject can be evaluated and determined to have pancreatic cancer.

The gene expression profile is a pattern of an expression signal such as fluorescence intensity, and recorded as a digital numerical value or as an image having colors. Comparison of gene expression profiles can be performed, for example, using a pattern comparison software, and a discriminant analysis, Cox hazard analysis, and the like can be used. A discriminant analysis model for evaluating and determining a pathological condition is constructed in advance, and data on the gene expression profile obtained from a subject are entered into the discriminant analysis model to evaluate and determine the pathological condition. For example, a discriminant formula is obtained by a discriminant analysis, and a fluorescence intensity is associated with a pathological condition. Then by assigning the numerical value of the expression signal of the subject to the discriminant formula, the pathological condition can be evaluated and determined.

The present invention provides a reagent for analyzing a gene expression profile comprising a nucleotide consisting of a nucleotide sequence of genes of the 56-gene set A of the present invention or a nucleotide comprising a partial sequence thereof and assist a diagnosis of pancreatic cancer by simultaneously measuring CA19-9 and introducing its value into the determination formula. The reagent for analyzing a gene expression profile is a reagent comprising a nucleotide consisting of a nucleotide sequence of said genes or a nucleotide comprising a partial sequence thereof as a primer or a probe, and a substrate such as a microarray which solidified a nucleotide consisting of a nucleotide sequence of said genes or a nucleotide comprising a partial sequence thereof. In the case of a PCR kit, it may contain a thermostable polymerase, a reverse transcriptase, a triphosphate deoxynucleotide, a triphosphate nucleotide, and the like.

The reagent for analyzing the gene expression profile comprises a nucleotide comprising a partial sequence of each of all 56 genes in the above 56-gene set A.

In the methods of the present invention, a discriminant formula for detecting whether a subject has pancreatic cancer or not can be constructed in advance, and by entering data on the expression level of the 56-gene set A and the measured value of CA19-9 obtained from the subject into the discriminant formula, whether the subject has pancreatic cancer or not can be detected. For example, a discriminant formula can be obtained by a discriminant analysis, and by assigning an expression level and a pathological condition to the discriminant formula and the expression level of the subject to the discriminant formula, the pathological condition can be evaluated and determined.

Examples of the numerical value for indicating the gene expression level include the number of cycles (Cycle threshold: Ct) when an amplified product reaches a certain amount when a target gene is amplified by real-time PCR. The number of cycles of amplification is plotted on the horizontal axis and the amount of PCR amplification product on the vertical axis to create an amplification curve. When a threshold value (Threshold) is set for the value of PCR amplification product, the cycle number at the intersection of the Threshold and the amplification curve is the Ct value. When a probe is used, a fluorescence intensity may also be used. When measuring the expression level, it is preferable to use a relative value to an expression level of a gene which is universally expressed at a constant level regardless of the condition of the subject. Examples of the gene which is universally expressed at a constant level include a housekeeping gene, such as glyceraldehyde 3-phosphate dehydrogenase (GAPDH). In the same sample, an expression level of GAPDH can be measured simultaneously with a measurement of an expression level of each gene as an internal standard, and the expression level of each gene can be indicated as a relative value to the expression level of GAPDH. For example, when measuring the expression level by real-time PCR, real-time PCR of a housekeeping gene (internal standard) with a constant expression level is performed to obtain the Ct value. Next, real-time PCR of a gene of interest is performed under the same conditions as for the housekeeping gene to obtain the Ct value. The Ct value of the gene of interest can be divided by the Ct value of the housekeeping gene to obtain a standardized Ct value, which can be used as a quantitative value of the gene for creating a discriminant formula. Alternatively, when the expression level of each gene is indicated by fluorescence intensity, the fluorescence intensity of each gene can be divided by the fluorescence intensity of GAPDH.

Discriminant analysis is a method of obtaining sample data extracted from 2 or more groups of people, and if there is a sample data wherein it is unknown which group it belongs to, finding out which group this sample data belongs to. In the present invention, a discriminant analysis is performed using multivariate data (x1, x2, ... xn) comprising numerical values related to expression levels of 56 or 47 genes in each of the 56-gene set A and 47-gene set B as explanatory variables.

In the methods of the present invention, real-time PCR can be used to measure the expression level and the following discriminant formula I can be used.

$$\text{Discriminant Formula I: intercept} + \sum (\text{beta i} \times X \text{ i})$$

wherein intercept is a constant, beta i is a coefficient for the i-th gene of the 56 genes in the gene set A, X i is the standardized Ct value of the i-th gene of the 56 genes in the gene set A, and $\Sigma$ indicates summing the beta $\times$ X of each gene of the 56 genes in the gene set A.

The Ct value of each gene in the 56-gene set A can be entered in the above formula I to calculate a numerical value. The calculated value is used in the following discriminant formula II as P.

Furthermore, the measured value of CA19-9 (unit is U/mL) is obtained and the final determination value Z is obtained using the following discriminant formula II.

$$\text{Discriminant Formula II: } -2.9448 + (0.0090864 \times P) + (0.9329593 \times \text{Loge C})$$

If the calculated final determination value Z is positive, i.e., the final determination value is greater than 0, it is determined positive for pancreatic cancer and if the value is negative, it is determined negative for pancreatic cancer. Here, that it is determined positive for pancreatic cancer means that it can be determined that the subject has pancreatic cancer.

Hereinafter, the values of intercept and beta of each gene from (1) to (56) in the 56-gene set A are shown. The value of beta for each gene in the gene set A is shown in Table 1.

Intercept for the gene set A: -298.018

[Table 1]

Set A

| No. | Gene name | beta | | No. | Gene name | beta |
|---|---|---|---|---|---|---|
| 1 | ABHD3 | 18.25234 | | 29 | IKBIP | -4.35974 |
| 2 | ABI1 | 5.533139 | | 30 | LDHA | 13.84082 |
| 3 | ACSL3 | -8.30246 | | 31 | LPAR6 | -11.9776 |
| 4 | AKR1B1 | 8.005092 | | 32 | MBTPS2 | 11.32147 |
| 5 | ATP 11B | -16.3131 | | 33 | MCMBP | 7.570634 |
| 6 | B3GNT5 | -12.6434 | | 34 | MIER1 | 4.196767 |
| 7 | BACH1 | 12.79754 | | 35 | NFE2L2 | 11.75957 |
| 8 | C11orf2 | -8.85243 | | 36 | OBFC2A | -11.0293 |
| 9 | C2orf81 | 8.747677 | | 37 | OSBPL8 | 6.956427 |
| 10 | CCNYL1 | 6.300126 | | 38 | PPIH | 5.40687 |
| 11 | CENPN | 4.732161 | | 39 | PPP1R13L | 8.60306 |
| 12 | CFHR3 | -6.547 | | 40 | PRDM5 | -8.77508 |
| 13 | CLEC4D | -7.20897 | | 41 | PTPRC | 15.18565 |
| 14 | COL17A1 | 7.970873 | | 42 | RAB 10 | 13.27343 |
| 15 | CYB5R4 | -11.661 | | 43 | RPLP1 | 13.75907 |
| 16 | DENND 1B | -10.4299 | | 44 | RRAGD | 6.236001 |
| 17 | ECHDC3 | -8.66068 | | 45 | SLC22A15 | -5.63841 |
| 18 | ENTPD1 | -7.19342 | | 46 | SLC44A1 | 18.88705 |
| 19 | FAM198B | -13.2931 | | 47 | SLFN12 | -7.8338 |
| 20 | FAM49B | -8.78217 | | 48 | SRBD1 | -12.4726 |
| 21 | FAR1 | 7.238576 | | 49 | TET2 | 11.42427 |
| 22 | FGL2 | -8.25242 | | 50 | TLE2 | 14.37697 |
| 23 | FNDC3B | 14.19903 | | 51 | UBE2W | 5.881697 |
| 24 | FPGT | -8.48534 | | 52 | UBE4A | 5.612217 |
| 25 | GALNT4 | 6.227974 | | 53 | USP15 | -7.21872 |
| 26 | HEATR5A | 5.784991 | | 54 | WSB1 | -13.4219 |
| 27 | HTATIP2 | 12.98543 | | 55 | ZEB2 | -8.42855 |
| 28 | IFNGR1 | -6.31219 | | 56 | ZMPSTE24 | 6.555173 |

Detection of pancreatic cancer according to the methods of the present invention is performed, for example, by the following step.

(1) mRNA is extracted from peripheral blood cells collected from a subject.
(2) The 56 genes in the gene set A are amplified by PCR to obtain a Ct (Cycle threshold) value. Then the Ct value is standardized by the Ct value of a housekeeping gene such as GAPDH to obtain the standardized Ct value.
(3) For the gene set A, the standardized Ct value of each gene in the gene set A is assigned to the discriminant formula I to obtain a P value.

$$\text{Discriminant formula I: intercept} + \Sigma \,(\text{beta } i \times X\, i)$$

wherein intercept is a constant, beta i is a coefficient for the i-th gene of the 56 genes in the gene set A, X i is the standardized Ct value of the i-th gene of the 56 genes in the gene set A, and $\Sigma$ indicates summing the beta $\times$ X of each gene of the 56 genes in the gene set A.

(4) CA19-9 in the biological sample collected from the subject is measured and set as a C value, and the C value and the P value in (3) are assigned to the discriminant formula II.

(5) A final determination value Z is obtained from the discriminant formula II = -2.9448 + (0.0090864 $\times$ P) + (0.9329593 $\times$ Loge C). If Z is positive, it is determined positive for pancreatic cancer and if Z is negative, it is determined negative for pancreatic cancer. Step (5) is a step for calculating a value to assist in determining whether the subject is positive or negative for pancreatic cancer by assigning the standardized Ct value and the measured value of CA19-9 for each gene in the gene set A to the discriminant formula.

The present invention comprises a system for detecting pancreatic cancer in a subject by the methods of detecting pancreatic cancer of the present invention.

The system of the present invention for detecting pancreatic cancer is a system comprising

(a) Data entry means for the gene expression profiles of the subject, wherein entered data for the gene expression profiles are, for example, the CA19-9 value measured simultaneously with data indicating the expression level of each gene, such as the standardized Ct value of each gene;

(b) Storage means storing the constructed discriminant formula; and

(c) Data processing means for determining a pathological condition of pancreatic cancer by applying the data entered using the data entry means in (a) to the discriminant formula stored in the storage means in (b).

Examples of the data entry means of (a) include a keyboard, or an external storage device which stores data. Examples of the storage means of (b) include a hard disk. The data processing means receives the discrimination model from the storage means, processes the entered data, and sends the result of the processing to an output means in which the result of the processing is displayed. Examples of the data processing means include a central processing unit (CPU) for arithmetic data processing. Examples of the output means include a monitor for displaying the result or a printer.

The system of the present invention can be constructed using a commercially available personal computer and the like.

Example

Hereinafter, the present invention is described specifically with reference to Example, although the present invention is not limited thereto.

Subj ects

Subjects used were 54 cases with pancreatic cancer, 22 cases with chronic pancreatitis, 25 cases with IPMN, and 103 healthy subjects.

Gene expression analysis

mRNA was extracted from peripheral blood cells collected from the subjects, and gene expression analysis of the 56 genes in the gene set A was performed by real-time PCR.

Collection of peripheral blood cells and extraction of RNA

Peripheral blood was collected from the subjects using PAXgene Blood RNA Tube (Nippon Becton Dickinson Company, Ltd., Medical device marketing authorization approval No.: 218AFBZX00014000).

Extraction of RNA and hybridization

RNA was extracted from PAXgene Blood RNA Tube using PAXgene Blood RNA Kit (PreAnalytiX GmbH, Hombrechtikon, Switzerland) according to the protocol.

Measurement of the amount of gene expression by real-time PCR

Expression analysis was performed by real-time PCR using the 56 genes in the gene set A.

For RNA, cDNA was synthesized using RNA as a template using High-Capacity cDNA Reverse Transcription Kit (Applied Biosystems, Foster City, CA, USA). An amplification reagent, a probe for selected a target gene and a probe for a housekeeping gene (18S ribosomal RNA) were mixed with the synthesized cDNA, and then the amount of the expression was measured by a multiplex PCR assay using Real Time PCR System Rotor-Gene Q (QIAGEN GmbH, Hilden, Germany).

The quantification method was that based on the quantified values of the target gene and the housekeeping gene calculated from the respective standard curve, the amount of the expression was calculated using Two Standard Curve method, in which the quantified value of the target gene was divided by the quantified value of the housekeeping gene, and the Ct value was standardized. Specifically, the 56 genes in the gene set A were amplified by PCR to obtain the Ct (Cycle threshold) value, and then using GAPDH as a housekeeping gene, standardized by the Ct value of said gene to obtain the standardized Ct value. Step from the reverse transcription to quantification of the amount of the expression was performed according to the QIAGEN protocol.

Measurement of CA19-9 (calculation of C value)

Serum was collected from the subjects and the measured value of CA19-9 was used using the following reagents and devices. The unit was U/mL. The obtained measured value was set as C.

Reagent: Chemilumi ACS Centaur CA19-9II

Device: ADVIA Centaur XP

Manufacturer: Siemens Healthcare Diagnostics K.K.

Measurement principle: Simultaneous measurement was performed by chemiluminescent immunoassay (CLIA method) to obtain the measured value.

Calculation by Discriminant Formula

Calculation of P value

The P value was obtained from the standardized Ct value of the 56 genes in the gene set A using the following discriminant formula I.

$$\text{Discriminant formula I: intercept} + \Sigma \, (\text{beta i} \times \text{X i})$$

wherein intercept is a constant, beta i is a coefficient for the i-th gene of the 56 genes in the gene set A, X i is the standardized Ct value of the i-th gene of the 56 genes in the gene set A, and $\Sigma$ indicates summing the beta $\times$ X of each gene of the 56 genes in the gene set A.

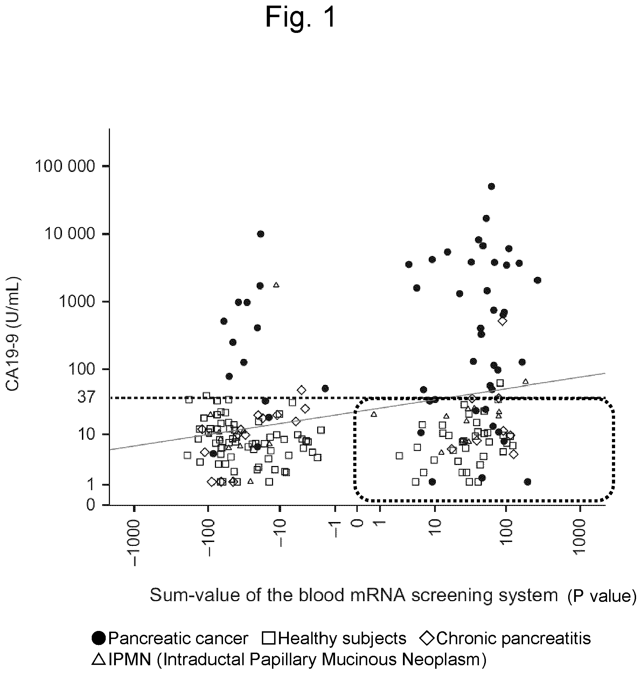

Figure 1 shows a correlation between C value (measured value of CA19-9) and P value (calculated value of discriminant formula based on the result of gene analysis).

Calculation of final determination value A

The measured value of CA19-9 was set as C, the calculated value of the gene set A by the discriminant formula I was set as P, and the final determination value Z was obtained by the following discriminant formula II.

$$\text{Discriminant formula II: } -2.9448 + (0.0090864 \times P) + (0.9329593 \times \text{Loge C})$$

If the final determination value Z is positive, it is determined to be positive for pancreatic cancer and if it is negative, it is determined to be negative.

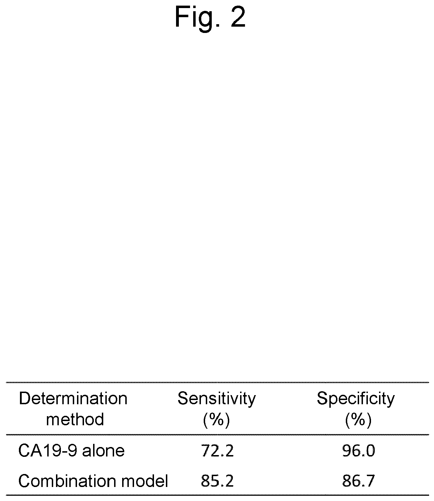

Figure 2 shows sensitivity and specificity in the case of detecting pancreatic cancer using CA19-9 alone and in the case of detecting pancreatic cancer using the measured value of CA19-9 in combination with the calculated value by 56 gene expression analysis. As shown by Figure 2, the sensitivity is higher in the case of using the values in combination.

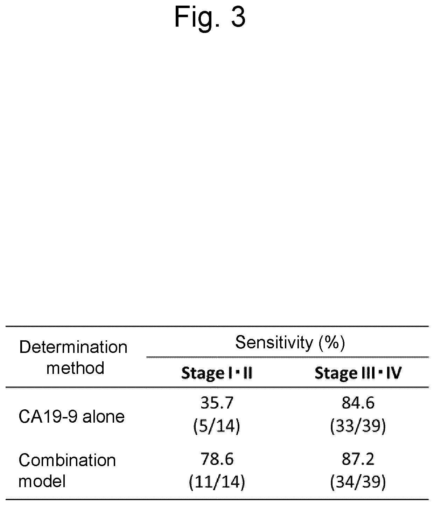

Figure 3 shows sensitivity by clinical stage (Stage I/II and Stage III/IV) in the case of detecting pancreatic cancer using CA19-9 alone and in the case of using the measured value of CA19-9 in combination with the calculated value by 56-gene expression analysis. As shown in Figure 3, the case using the values in combination shows higher sensitivity in both the early stages of Stage I/II and in the advanced stages of Stage III/IV, in particular with significantly higher sensitivity in the early stages than the case of using CA19-9 alone.

Industrial Applicability

The present invention enables a minimally invasive, simple, and highly accurate detection of pancreatic cancer.

All publications, patents and patent applications cited herein are incorporated herein by reference in their entirety.

**Claims**

1. A method for assisting detection of pancreatic cancer based on an expression level of 56 genes in a 56-gene set A listed below and a measured value of CA19-9 in a subject:

   the gene set A being

   (1) Abhydrolase domain containing 3 (ABHD3)
   (2) Abl-interactor 1 (ABI1)
   (3) Acyl-CoA synthetase long-chain family member 3 (ACSL3)
   (4) Aldo-keto reductase family 1, member B1 (aldose reductase) (AKR1B 1)
   (5) ATPase, Class VI, type 11B (ATP11B)
   (6) UDP-GlcNAc:betaGal beta-1,3-N-acetylglucosaminyltransferase 5 (B3GNT5)
   (7) BTB and CNC homology 1, basic leucine zipper transcription factor 1 (BACH1)
   (8) Chromosome 11 open reading frame 2 (C11orf2)
   (9) Chromosome 2 open reading frame 81 (C2orf81)
   (10) Cyclin Y-like 1 (CCNYL1)
   (11) Centromere protein N (CENPN)
   (12) Complement factor H-related 3 (CFHR3)
   (13) C-type lectin domain family 4, member D (CLEC4D)
   (14) Collagen, type XVII, alpha 1 (COL17A1)
   (15) Cytochrome b5 reductase 4 (CYB5R4)
   (16) DENN/MADD domain containing 1B (DENND1B)
   (17) Enoyl CoA hydratase domain containing 3 (ECHDC3)
   (18) Ectonucleoside triphosphate diphosphohydrolase 1 (ENTPD1)
   (19) Family with sequence similarity 198, member B (FAM198B)
   (20) Family with sequence similarity 49, member B (FAM49B)
   (21) Fatty acyl CoA reductase 1 (FAR1)
   (22) Fibrinogen-like 2 (FGL2)
   (23) Fibronectin type III domain containing 3B (FNDC3B)
   (24) Fucose-1-phosphate guanylyltransferase (FPGT)
   (25) UDP-N-acetyl-alpha-D-galactosamine:polypeptide N-acetylgalactosaminyltransferase 4 (GalNAc-T4) (GALNT4)
   (26) HEAT repeat containing 5A (HEATR5A)
   (27) HIV-1 Tat interactive protein 2, 30kDa (HTATIP2)
   (28) Interferon gamma receptor 1 (IFNGR1)
   (29) IKBKB interacting protein (IKBIP)
   (30) Lactate dehydrogenase A (LDHA)
   (31) Lysophosphatidic acid receptor 6 (LPAR6)
   (32) Membrane-bound transcription factor peptidase, site 2 (MBTPS2)
   (33) Minichromosome maintenance complex binding protein (MCMBP)
   (34) Mesoderm induction early response 1 homolog (Xenopus laevis) (MIER1)
   (35) Nuclear factor (erythroid-derived 2) -like 2 (NFE2L2)
   (36) Oligonucleotide/oligosaccharide-binding fold containing 2A (OBFC2A)
   (37) Oxysterol binding protein-like 8 (OSBPL8)
   (38) Peptidylprolyl isomerase H (cyclophilin H) (PPIH)

(39) Protein phosphatase 1, regulatory (inhibitor) subunit 13 like (PPP1R13L)

(40) PR domain containing 5 (PRDM5)

(41) Protein tyrosine phosphatase, receptor type, C (PTPRC)

(42) RAB10, member RAS oncogene family (RAB10)

(43) Ribosomal protein, large, P1 (RPLP1)

(44) Ras-related GTP binding D (RRAGD)

(45) Solute carrier family 22, member 15 (SLC22A15)

(46) Solute carrier family 44, member 1 (SLC44A1)

(47) Schlafen family member 12 (SLFN12)

(48) S1 RNA binding domain 1 (SRBD1)

(49) Tet oncogene family member 2 (TET2)

(50) Transducin-like enhancer of split 2 (E(sp1) homolog, Drosophila) (TLE2)

(51) Ubiquitin-conjugating enzyme E2W (putative) (UBE2W)

(52) Ubiquitination factor E4A (UBE4A)

(53) Ubiquitin specific peptidase 15 (USP15)

(54) WD repeat and SOCS box containing 1 (WSB1)

(55) Zinc finger E-box binding homeobox 2 (ZEB2)

(56) Zinc metallopeptidase (STE24 homolog, S. cerevisiae) (ZMPSTE24)

2. The method according to claim 1, wherein the expression level of the genes in the subject is measured using mRNA of peripheral blood cells of the subject.

3. The method according to claim 1, wherein the gene expression level is measured by quantitative PCR targeting a nucleotide consisting of a nucleotide sequence of the 56 genes in the 56-gene set A defined in claim 1 or a nucleotide comprising a partial nucleotide sequence thereof, and used in combination with the measured value of CA19-9.

4. The method according to any one of claims 1 to 3, comprising steps below:

(1) a step of extracting mRNA from the peripheral blood cells collected from the subject;
(2) a step of amplifying the 56 genes in the gene set A by PCR to obtain a Ct (Cycle threshold) value, and standardizing it with a Ct value of a housekeeping gene such as GAPDH to obtain a standardized Ct value;
(3) a step of measuring CA19-9 in a biological sample collected from the subject;
(4) a step of calculating a value for assisting in determining whether it is positive or negative for pancreatic cancer from the standardized Ct Value of each gene in the gene set A and the measured value of CA19-9;

5. The method according to claim 4, comprising steps below wherein if a final determination value Z is greater than 0, it is determined positive for pancreatic cancer:

(1) a step of calculating a P value from the standardized Ct value obtained in claim 4 step (2) using a discriminant formula I described below,

$$\text{Discriminant formula I: intercept} + \sum (\text{beta } i \times X\, i)$$

wherein intercept is a constant, beta i is a coefficient for the i-th gene of the 56 genes in the gene set A, and x i indicates summing beta × X for each of the 56 genes in the gene set A; and
(2) a step of setting the measured value of CA19-9 obtained in claim 4 step (3) as a C value and assigning the P value and the C value to a discriminant formula II described below to obtain a final determination value Z,

$$\text{Discriminant formula II: } -2.9448 + (0.0090864 \times P) + (0.9329593 \times \text{Loge } C).$$

6. The method for detecting pancreatic cancer according to claim 5, wherein the values of intercept and beta i for each gene in the discriminant formula using the 56-gene set A are as follows:

intercept: -298.018
Set A

| No. | Gene name | beta |
|---|---|---|
| 1 | ABHD3 | 18.25234 |
| 2 | ABI1 | 5.533139 |
| 3 | ACSL3 | -8.30246 |
| 4 | AKR1B1 | 8.005092 |
| 5 | ATP 11B | -16.3131 |
| 6 | B3GNT5 | -12.6434 |
| 7 | BACH1 | 12.79754 |
| 8 | C11orf2 | -8.85243 |
| 9 | C2orf81 | 8.747677 |
| 10 | CCNYL1 | 6.300126 |
| 11 | CENPN | 4.732161 |
| 12 | CFHR3 | -6.547 |
| 13 | CLEC4D | -7.20897 |
| 14 | COL17A1 | 7.970873 |
| 15 | CYB5R4 | -11.661 |
| 16 | DENND 1B | -10.4299 |
| 17 | ECHDC3 | -8.66068 |
| 18 | ENTPD1 | -7.19342 |
| 19 | FAM198B | -13.2931 |
| 20 | FAM49B | -8.78217 |
| 21 | FAR1 | 7.238576 |
| 22 | FGL2 | -8.25242 |
| 23 | FNDC3B | 14.19903 |
| 24 | FPGT | -8.48534 |
| 25 | GALNT4 | 6.227974 |
| 26 | HEATR5A | 5.784991 |
| 27 | HTATIP2 | 12.98543 |
| 28 | IFNGR1 | -6.31219 |
| 29 | IKBIP | -4.35974 |
| 30 | LDHA | 13.84082 |
| 31 | LPAR6 | -11.9776 |
| 32 | MBTPS2 | 11.32147 |
| 33 | MCMBP | 7.570634 |
| 34 | MIER1 | 4.196767 |
| 35 | NFE2L2 | 11.75957 |
| 36 | OBFC2A | -11.0293 |
| 37 | OSBPL8 | 6.956427 |

17

(continued)

| No. | Gene name | beta |
|-----|-----------|------|
| 38 | PPIH | 5.40687 |
| 39 | PPP1R13L | 8.60306 |
| 40 | PRDM5 | -8.77508 |
| 41 | PTPRC | 15.18565 |
| 42 | RAB10 | 13.27343 |
| 43 | RPLP1 | 13.75907 |
| 44 | RRAGD | 6.236001 |
| 45 | SLC22A15 | -5.63841 |
| 46 | SLC44A1 | 18.88705 |
| 47 | SLFN12 | -7.8338 |
| 48 | SRBD1 | -12.4726 |
| 49 | TET2 | 11.42427 |
| 50 | TLE2 | 14.37697 |
| 51 | UBE2W | 5.881697 |
| 52 | UBE4A | 5.612217 |
| 53 | USP15 | -7.21872 |
| 54 | WSB1 | -13.4219 |
| 55 | ZEB2 | -8.42855 |
| 56 | ZMPSTE24 | 6.555173 |

7. A reagent for detecting pancreatic cancer, comprising a nucleotide consisting of a nucleotide sequence of the genes in the 56-gene set A or a nucleotide comprising a partial nucleotide sequence thereof for detecting pancreatic cancer by use in combination with the measurement of CA 19-9:
the gene set A being

(1) Abhydrolase domain containing 3 (ABHD3)
(2) Abl-interactor 1 (ABI1)
(3) Acyl-CoA synthetase long-chain family member 3 (ACSL3)
(4) Aldo-keto reductase family 1, member B1 (aldose reductase) (AKR1B 1)
(5) ATPase, Class VI, type 11B (ATP11B)
(6) UDP-GlcNAc:betaGal beta-1,3-N-acetylglucosaminyltransferase 5 (B3GNT5)
(7) BTB and CNC homology 1, basic leucine zipper transcription factor 1 (BACH1)
(8) Chromosome 11 open reading frame 2 (C11orf2)
(9) Chromosome 2 open reading frame 81 (C2orf81)
(10) Cyclin Y-like 1 (CCNYL1)
(11) Centromere protein N (CENPN)
(12) Complement factor H-related 3 (CFHR3)
(13) C-type lectin domain family 4, member D (CLEC4D)
(14) Collagen, type XVII, alpha 1 (COL17A1)
(15) Cytochrome b5 reductase 4 (CYB5R4)
(16) DENN/MADD domain containing 1B (DENND1B)
(17) Enoyl CoA hydratase domain containing 3 (ECHDC3)
(18) Ectonucleoside triphosphate diphosphohydrolase 1 (ENTPD1)
(19) Family with sequence similarity 198, member B (FAM198B)
(20) Family with sequence similarity 49, member B (FAM49B)
(21) Fatty acyl CoA reductase 1 (FAR1)

(22) Fibrinogen-like 2 (FGL2)

(23) Fibronectin type III domain containing 3B (FNDC3B)

(24) Fucose-1-phosphate guanylyltransferase (FPGT)

(25) UDP-N-acetyl-alpha-D-galactosamine:polypeptide N-acetylgalactosaminyltransferase 4 (GalNAc-T4) (GALNT4)

(26) HEAT repeat containing 5A (HEATR5A)

(27) HIV-1 Tat interactive protein 2, 30kDa (HTATIP2)

(28) Interferon gamma receptor 1 (IFNGR1)

(29) IKBKB interacting protein (IKBIP)

(30) Lactate dehydrogenase A (LDHA)

(31) Lysophosphatidic acid receptor 6 (LPAR6)

(32) Membrane-bound transcription factor peptidase, site 2 (MBTPS2)

(33) Minichromosome maintenance complex binding protein (MCMBP)

(34) Mesoderm induction early response 1 homolog (Xenopus laevis) (MIER1)

(35) Nuclear factor (erythroid-derived 2) -like 2 (NFE2L2)

(36) Oligonucleotide/oligosaccharide-binding fold containing 2A (OBFC2A)

(37) Oxysterol binding protein-like 8 (OSBPL8)

(38) Peptidylprolyl isomerase H (cyclophilin H) (PPIH)

(39) Protein phosphatase 1, regulatory (inhibitor) subunit 13 like (PPP1R13L)

(40) PR domain containing 5 (PRDM5)

(41) Protein tyrosine phosphatase, receptor type, C (PTPRC)

(42) RAB10, member RAS oncogene family (RAB10)

(43) Ribosomal protein, large, P1 (RPLP1)

(44) Ras-related GTP binding D (RRAGD)

(45) Solute carrier family 22, member 15 (SLC22A15)

(46) Solute carrier family 44, member 1 (SLC44A1)

(47) Schlafen family member 12 (SLFN12)

(48) S1 RNA binding domain 1 (SRBD1)

(49) Tet oncogene family member 2 (TET2)

(50) Transducin-like enhancer of split 2 (E(sp1) homolog, Drosophila) (TLE2)

(51) Ubiquitin-conjugating enzyme E2W (putative) (UBE2W)

(52) Ubiquitination factor E4A (UBE4A)

(53) Ubiquitin specific peptidase 15 (USP15)

(54) WD repeat and SOCS box containing 1 (WSB1)

(55) Zinc finger E-box binding homeobox 2 (ZEB2)

(56) Zinc metallopeptidase (STE24 homolog, S. cerevisiae) (ZMPSTE24)

**8.** The reagent according to claim 7, wherein the nucleotide comprising the partial nucleotide sequence is a primer for PCR.

# Fig. 1

Sum-value of the blood mRNA screening system (P value)

●Pancreatic cancer  □Healthy subjects  ◇Chronic pancreatitis
△IPMN (Intraductal Papillary Mucinous Neoplasm)

Fig. 2

| Determination method | Sensitivity (%) | Specificity (%) |
|---|---|---|
| CA19-9 alone | 72.2 | 96.0 |
| Combination model | 85.2 | 86.7 |

# Fig. 3

| Determination method | Sensitivity (%) | |
|---|---|---|
| | Stage I · II | Stage III · IV |
| CA19-9 alone | 35.7 (5/14) | 84.6 (33/39) |
| Combination model | 78.6 (11/14) | 87.2 (34/39) |

**INTERNATIONAL SEARCH REPORT**

| International application No. |
| --- |
| **PCT/JP2022/033204** |

**A. CLASSIFICATION OF SUBJECT MATTER**

*C12N 15/09*(2006.01)i; *C12Q 1/68*(2018.01)i; *C12Q 1/6851*(2018.01)i; *C12Q 1/6886*(2018.01)i
FI: C12Q1/68 ZNA; C12Q1/6851; C12Q1/6886; C12N15/09 Z

According to International Patent Classification (IPC) or to both national classification and IPC

**B. FIELDS SEARCHED**

Minimum documentation searched (classification system followed by classification symbols)

C12N15/09; C12Q1/68; C12Q1/6851; C12Q1/6886

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| Y | 酒井佳夫、金子周一, 膵癌の新たな血液バイオマーカーに対する前向き医師主導臨床性能試験, 日本消化器病学会雑誌, 2020, vol. 117, extraordinary special issue, page A63, S9-13, (SAKAI, Yoshio, KANEKO, Shuichi.), non-official translation (Prospective, investigator-initiated clinical performance test for novel blood biomarkers in pancreatic cancer. Journal of Japan Society of Gastroenterology.)<br>entire text | 1-8 |
| Y | JP 2016-192944 A (KUBIX INC.) 17 November 2016 (2016-11-17)<br>claims, examples | 1-8 |
| Y | SAKAI, Yoshio et al. Development of novel diagnostic system for pancreatic cancer including early stages, measuring mRNA of whole blood cells. Cancer Sci. 2019, vol. 110, pages 1364-1388<br>table 1 | 1-8 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **10 November 2022** | **22 November 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2022/033204** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "The form of Annex C/ST.25 text file" above shall read as "the form of ST.26.".

Form PCT/ISA/210 (continuation of first sheet) (January 2015)

INTERNATIONAL SEARCH REPORT
Information on patent family members

International application No.

**PCT/JP2022/033204**

| Patent document cited in search report | | | Publication date (day/month/year) | Patent family member(s) | Publication date (day/month/year) |
|---|---|---|---|---|---|
| JP | 2016-192944 | A | 17 November 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 37677 A
- JP 5852759 B
- US 527154 A
- US 544861 A
- EP 0761822 A

### Non-patent literature cited in the description

- **KATO, K. et al.** *Nucl. Acids Res.,* 1997, vol. 25, 4694-4696
- **SCHMITTGEN TD.** *Methods,* 2001, vol. 25, 383-385
- Body Map method. *Gene,* 1996, vol. 174, 151-158